# EUROPEAN PATENT APPLICATION

(11) **EP 3 182 309 A1**
(43) Date of publication of application: **21.06.2017**
(21) Application number: 15201007.0
(22) Date of filing: 18.12.2015
(51) Int. Cl.: G06F 19/00, G06F 17/30

(54) **METHOD OF CREATING A UNIQUE PATIENT REFERENCE**

(71) Applicant: Agfa Healthcare, 2640 Mortsel (BE)
(72) Inventor: Sun, Hong, 2640 Mortsel (BE); De Roo, Jos, 2640 Mortsel (BE); Depraetere, Kristof, 2640 Mortsel (BE); Colaert, Dirk, 2640 Mortsel (BE)
(74) Representative: Verbrugghe, Anne Marie L.

(57) **Abstract**

A method to link instances expressed in at least two EHR systems by means of URIs constructed with different identifier sets by processing RDF data of said instances to link the instances by generating re-labeling statements and applying the re-labeling statements to the RDF data to consolidate the URIs with different identifier types to a single URI with a master identity.

## Description

### FIELD OF THE INVENTION

The present invention is in the field of semantic web technology. More specifically the invention relates to semantic web technology applied in the context of electronic health records.

The invention relates to a method of generating a unique patient reference in case patient data used in a certain system originate from different electronic health record systems (EHR) using different types of identifiers.

### BACKGROUND OF THE INVENTION

A patient may have different identifiers when the patient's data is stored in different EHR systems.

For example Agfa HealthCare has developed two EHR systems named ORBIS and IMPAX respectively in which patient data are identified by means of different identifiers.

This may cause problems when patient data originating from such different systems are to be merged.

A so-called Master Patient Identity (MPI) exists which is a unique identifier used to refer to a patient across an enterprise or across different EHR systems. An MPI can be assigned to each patient and refers to different patient characteristics such as name, date of birth, gender etc. This MPI may for example be stored in the patient's electronic identification card and is a unique identifier which in many cases is not the identifier that is used in local EHR systems.

The use of this Master Patient Identity (MPI) could help to merge patient data from different systems. However, even then the following challenges still remain to be considered and solved.

It is considered convenient to use a local identity identifier in a data storage system such as an EHR to create a patient reference inside a local system, where tables are joined using local identities, and indexes are also built on local identities. Creating a patient reference by means of a master identity might create a performance and reusability issue.

Furthermore when in a certain EHR system a patient reference is used which is created by means of a local identity, the implication of master identity might be complex.

Still further, the problem of unique identifiers is not restricted to patient identity identifier, but also applies to identifiers of healthcare providers, medical cases, lab requests, etc.

In the context of a semantic data representation there is thus a need to provide a method to identify Resource Description Framework (RDF) instances in a unique way across systems, more particularly across EHR systems, that locally apply different data identifiers.

It is an aspect of the present invention to provide such a method.

### SUMMARY OF THE INVENTION

The above-mentioned aspect is realized by a method having the specific method steps set out in claim 1.

In the context of the present invention the following references are made.

RDF data refers to Resource Description Framework data which is a semantic metadata model used for data interchange.

URI refers to a resource identification on the world wide web.

The term 'local identifier' refers to an identifier for identifying an instance which is used locally in a specific electronic health record system and which is specific for such system.

Master identity refers to an identifier of an instance which is general across health record systems.

Specific features for preferred embodiments of the invention are set out in the dependent claims.

Further advantages and embodiments of the present invention will become apparent from the following description.

The present invention relates to an approach that uses local identity references (specific for a given EHR system) to create a patient reference. Master identity related information is retrieved and processed to generate a set of re-label statements that re-label the local identity reference to a new reference represented by means of the master identity.

The re-label statements are only applied when needed, which guarantees high performance of the system.

The solution of the present invention not only applies to patient identifiers, but also to other types of identifiers such as identifiers of healthcare providers, medical cases, lab requests, etc.

### BRIEF DESCRIPTION OF THE DRAWINGS

Fig. 1 shows the visualized RDF graph presented in Table 4, it links two instances with owl:sameAs.
Fig. 2 shows the visualized RDF graph presented in Table 6, it applied the entailment rules of owl:sameAs (Table 5) to the graph shown in Fig. 1.
Fig. 3 shows the visualized RDF graph presented in Table 7, it uses MPI to replace the local identifiers.
Fig. 4 shows a sample data structure for identifiers.

### DETAILED DESCRIPTION OF THE INVENTION

Suppose the following data coming from two systems inside a same hospital, where a patient has two different patient identifications (IDs) in these two systems, as well as a common master identity.

For example Agfa HealthCare has been marketing an EHR system named ORBIS as well as an image archiving system named IMPAX. Both systems use different patient identifiers for a same patient.

The present invention will be explained below with reference to patient identification in each of these systems of Agfa HealthCare.

In the ORBIS system a patient is identified by means of a first local ID of the ORBIS system as 10101.

In the image archiving system IMPAX (used in a radiographic environment) a patient is identified by means of a local ID of the IMPAX system as 20202.

Furthermore in both systems this patient instance is identified by means of a master ID as 8800123, this master ID is general, i.e. the same across systems.

Tables 1 and 2 illustrate sample patient graphs regarding the same patient in Agfa HealthCare's ORBIS system and Agfa HealthCare's IMPAX system respectively.

**Table 1. sample patient graph-orbis**

| |
|---|
| <https://agfa.com/orbis/resource/Patient/10101> |
| <http://www.w3.org/1999/02/22-rdf-syntax-ns#type> |
| <http://purl.bioontology.org/ontology/SNOMEDCT/116154003>. |
| <https://agfa.com/orbis/resource/Patient/10101> |
| <http://aca.agfa.net/resource/ontology/healthcare#hasExamination> |
| <https://agfa.com/orbis/resource/LabResult/2454564>. |
| <https://agfa.com/orbis/resource/Patient/10101> |
| <http://schema.org/identifier> 8800123 . |
| <https://agfa.com/orbis/resource/LabResult/2454564> |
| <http://aca.agfa.net/resource/ontology/healthcare#medicalCase> |
| <https://agfa.com/orbis/resource/Fall/12345>. |
| <https://agfa.com/orbis/resource/LabResult/2454564> |
| <http://schema.org/value> 9.60 . |

**Table 2. sample patient graph-impax**

| |
|---|
| <https://agfa.com/impax/resource/Patient/20202> |
| <http://www.w3.org/1999/02/22-rdf-syntax-ns#type> |
| <http://purl.bioontology.org/ontology/SNOMEDCT/116154003>. |
| <https://agfa.com/impax/resource/Patient/20202> |
| <http://schema.org/identifier> 8800123 . |
| <https://agfa.com/impax/resource/Patient/20202> |
| <http://aca.agfa.net/resource/ontology/healthcare#hasExamination> |
| <https://agfa.com/impax/resource/Exam/12345>. |
| <https://agfa.com/impax/resource/Exam/12345> |
| <http://schema.org/value> 1.23 . |

A conventional way to link the two graphs of this same patient, with the semantic web technology, is to create a conversion rule so as to deduce that <https://agfa.com/orbis/resource/Patient/10101> and <https://agfa.com/impax/resource/Patient/20202> are references to the same patient.

**Table 3. sample master identity conversion rule**

| |
|---|
| ```
{    ?patient1 <http://schema.org/identifier> ?mpi .
     ?patient2 <http://schema.org/identifier> ?mpi .
     ?patient1 log:notEqualTo ?patient2 .
} => {
     ?patient1 owl:sameAs ?patient2.
}
``` |

Apply this master identity conversion rule on the above data results in a mixed reference as illustrated in table 4.

**Table 4. mixed reference**

| |
|---|
| <https://agfa.com/orbis/resource/Patient/10101> |
| <http://www.w3.org/1999/02/22-rdf-syntax-ns#type> |
| <http://purl.bioontology.org/ontology/SNOMEDCT/116154003>. |
| <https://agfa.com/orbis/resource/Patient/10101> |
| <http://aca.agfa.net/resource/ontology/healthcare#hasExamination> |
| <https://agfa.com/orbis/resource/LabResult/2454564>. |
| <https://agfa.com/orbis/resource/Patient/10101> |
| <http://schema.org/identifier> 8800123 . |
| <https://agfa.com/orbis/resource/LabResult/2454564> |
| <http://aca.agfa.net/resource/ontology/healthcare#medicalCase> |
| <https://agfa.com/orbis/resource/Fall/12345>. |
| <https://agfa.com/orbis/resource/LabResult/2454564> |
| <http://schema.org/value> 9.60 . |
| |
| <https://agfa.com/impax/resource/Patient/20202> |
| <http://www.w3.org/1999/02/22-rdf-syntax-ns#type> |
| <http://purl.bioontology.org/ontology/SNOMEDCT/116154003>. |
| <https://agfa.com/impax/resource/Patient/20202> |
| <http://schema.org/identifier> 8800123 . |
| <https://agfa.com/impax/resource/Patient/20202> |
| <http://aca.agfa.net/resource/ontology/healthcare#hasExamination> |
| <https://agfa.com/impax/resource/Exam/12345>. |
| <https://agfa.com/impax/resource/Exam/12345> |
| <http://schema.org/value> 1.23 . |
| |
| <https://agfa.com/orbis/resource/Patient/10101> owl:sameAs |
| <https://agfa.com/impax/resource/Patient/20202>. |

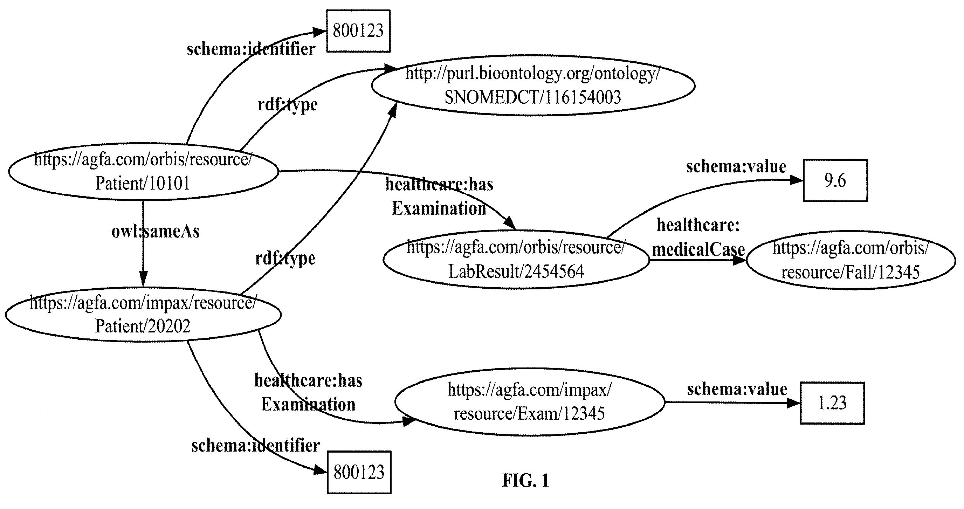

However, such expression still need to solve the entailment of owl:sameAs when it is in use, as it can be observed from the visual graph representation shown in figure 1:

**Table 5: OWL sameAs entailment rule**

| |
|---|
| @prefix owl: <http://www.w3.org/2002/07/owl#>. |
| |
| {?X owl:sameAs ?Y} => {?Y owl:sameAs ?x}. |
| {?X owl:sameAs ?Y. ?Y owl:sameAs ?Z} => {?X owl:sameAs ?Z}. |
| {?X owl:sameAs ?Y. ?X ?P ?O} => {?Y ?P ?O}. |
| {?X owl:sameAs ?Y. ?S ?X ?O} => {?S ?Y ?O}. |
| {?X owl:sameAs ?Y. ?S ?P ?X} => {?S ?P ?Y}. |

Table 5 shows the entailments brought by owl:sameAs, expressed as N3 rules. Applying the Rules in Table 5 to the mixed data in Table 4, it yields entailed results as displayed in Table 6.

**Table 6: Entailed results**

| |
|---|
| #Processed by EYE-Autumn15 10191509Z josd |
| #eye --nope mixed.ttl owl-inference.n3 --pass |
| PREFIX owl: <http://www.w3.org/2002/07/owl#> |
| PREFIX log: <http://www.w3.org/2000/10/swap/log#> |
| PREFIX rdfs: <http://www.w3.org/2000/01/rdf-schema#> |
| PREFIX rdf: <http://www.w3.org/1999/02/22-rdf-syntax-ns#> |
| |
| <https://agfa.com/orbis/resource/Patient/10101> a |
| <http://purl.bioontology.org/ontology/SNOMEDCT/116154003>. |
| <https://agfa.com/impax/resource/Patient/20202> a |
| <http://purl.bioontology.org/ontology/SNOMEDCT/116154003>. |
| <https://agfa.com/orbis/resource/Patient/10101> |
| <http://aca.agfa.net/resource/ontology/healthcare#hasExamination> |
| <https://agfa.com/orbis/resource/LabResult/2454564>. |
| <https://agfa.com/impax/resource/Patient/20202> |
| <http://aca.agfa.net/resource/ontology/healthcare#hasExamination> |
| <https://agfa.com/impax/resource/Exam/12345>. |
| <https://agfa.com/impax/resource/Patient/20202> |
| <http://aca.agfa.net/resource/ontology/healthcare#hasExamination> |
| <https://agfa.com/orbis/resource/LabResult/2454564>. |
| <https://agfa.com/orbis/resource/Patient/10101> |
| <http://aca.agfa.net/resource/ontology/healthcare#hasExamination> |
| <https://agfa.com/impax/resource/Exam/12345>. |
| <https://agfa.com/orbis/resource/Patient/10101> |
| <http://schema.org/identifier> 8800123 . |
| <https://agfa.com/impax/resource/Patient/20202> |
| <http://schema.org/identifier> 8800123 . |
| <https://agfa.com/orbis/resource/LabResult/2454564> |
| <http://aca.agfa.net/resource/ontology/healthcare#medicalCase> |
| <https://agfa.com/orbis/resource/Fall/12345>. |
| <https://agfa.com/orbis/resource/LabResult/2454564> |
| <http://schema.org/value> 9.6 . |
| <https://agfa.com/impax/resource/Exam/12345> |
| <http://schema.org/value> 1.23 . |
| <https://agfa.com/orbis/resource/Patient/10101> owl:sameAs |
| <https://agfa.com/impax/resource/Patient/20202>. |
| <https://agfa.com/impax/resource/Patient/20202> owl:sameAs |
| <https://agfa.com/orbis/resource/Patient/10101>. |
| <https://agfa.com/orbis/resource/Patient/10101> owl:sameAs |
| <https://agfa.com/orbis/resource/Patient/10101>. |
| <https://agfa.com/impax/resource/Patient/20202> owl:sameAs |
| <https://agfa.com/impax/resource/Patient/20202>. |
| #ENDS 0.078 [sec] IO=15/15 TC=5 TP=79 BC=0 BP=0 PM=0 CM=0 FM=0 AM=0 |

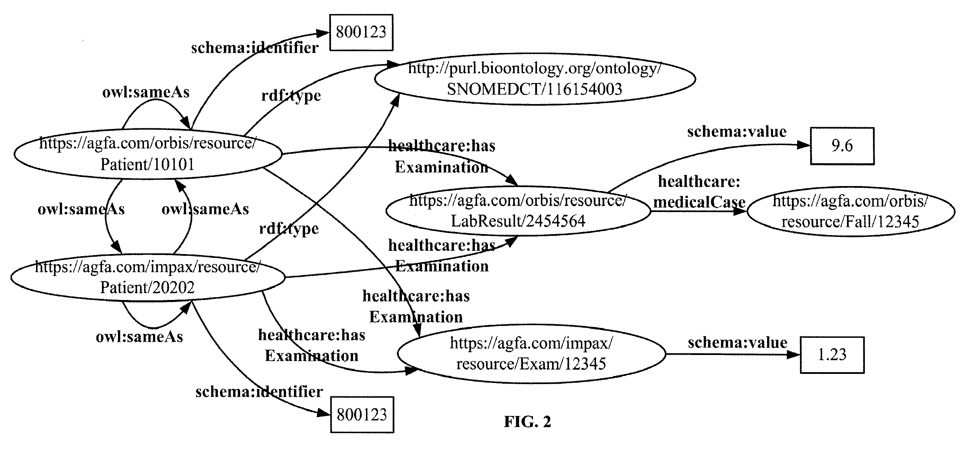

The figure below (Fig. 2) visualizes the data in Table 6.

The problem can be tackled if data from both ORBIS and IMPAX uses master identity to construct the reference for a patient. Then, the sample master identity rule listed in Table 3 is not needed, and the data would be merged as:

**Table 7. use mpi as reference**

| |
|---|
| <https://agfa.com/resource/Patient/8800123> |
| <http://www.w3.org/1999/02/22-rdf-syntax-ns#type> |
| <http://purl.bioontology.org/ontology/SNOMEDCT/116154003> . |
| <https://agfa.com/resource/Patient/8800123> |
| <http://aca.agfa.net/resource/ontology/healthcare#hasExamination> |
| <https://agfa.com/orbis/resource/LabResult/2454564>. |
| <https://agfa.com/resource/Patient/8800123> |
| <http://schema.org/identifier> 8800123 . |
| <https://agfa.com/orbis/resource/LabResult/2454564> |
| <http://aca.agfa.net/resource/ontology/healthcare#medicalCase> |
| <https://agfa.com/orbis/resource/Fall/12345>. |
| <https://agfa.com/orbis/resource/LabResult/2454564> |
| <http://schema.org/value> 9.60 . |
| |
| <https://agfa.com/resource/Patient/8800123> |
| <http://www.w3.org/1999/02/22-rdf-syntax-ns#type> |
| <http://purl.bioontology.org/ontology/SNOMEDCT/116154003> . |
| <https://agfa.com/resource/Patient/8800123> |
| <http://aca.agfa.net/resource/ontology/healthcare#hasExamination> |
| <https://agfa.com/impax/resource/Exam/12345>. |
| <https://agfa.com/impax/resource/Exam/12345> |
| <http://schema.org/value> 1.23 . |

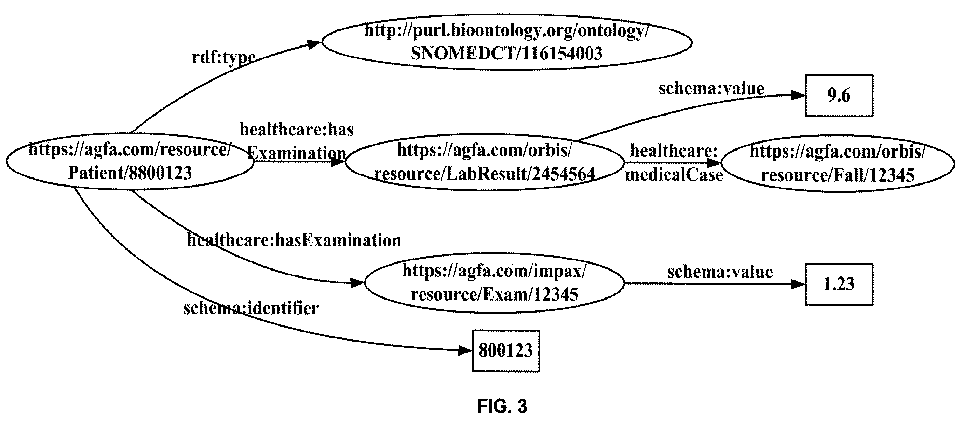

It can be observed from the visual graph representation (Fig. 3) below that no further inference is required compared with the previous graph represented in Table 4.

The present invention allows different systems to use a reference as is convenient in the local system, and yet use a master identity re-label process to replace local ID references with expressions using the master identity.

In this way each system processes the data in its particular way, conventional to the particular system. For example, both ORBIS and IMPAX can generate their data as the graphs shown in Table 1 and Table 2.

Instead of using the master identity conversion rule in Table 3 to link the two graphs, a set of re-labeling statements is used.

This set of re-labeling statements is e.g. for ORBIS

**Table 8. ORBIS re-labeling statements**

| |
|---|
| <https://agfa.com/orbis/resource/Patient/10101> e:relabel |
| <https://agfa.com/resource/Patient/8800123>. |
| <https://agfa.com/orbis/resource/Patient/10102> e:relabel |
| <https://agfa.com/resource/Patient/8800124>. |
| <https://agfa.com/orbis/resource/Patient/10103> e:relabel |
| <https://agfa.com/resource/Patient/8800125>. |
| --- |
| |
| e.g. for IMPAX |

**Table 9. IMPAX re-labeling statements**

| |
|---|
| <https://agfa.com/impax/resource/Patient/20202> e:relabel |
| <https://agfa.com/resource/Patient/8800123>. |
| <https://agfa.com/impax/resource/Patient/20203> e:relabel |
| <https://agfa.com/resource/Patient/8800124>. |
| <https://agfa.com/impax/resource/Patient/20204> e:relabel |
| <https://agfa.com/resource/Patient/8800125>. |
| ... |

By applying the rules in table 8 and 9 to the data in table 1 and 2, the local references may be replaced by a global reference. By putting the re-labeled graphs together, the graph expressed in Table 7 can be retrieved.

### Implementations

The relabeling statements can be generated either with a query rule, expressed with N3 logics, or directly via SQL scripts.

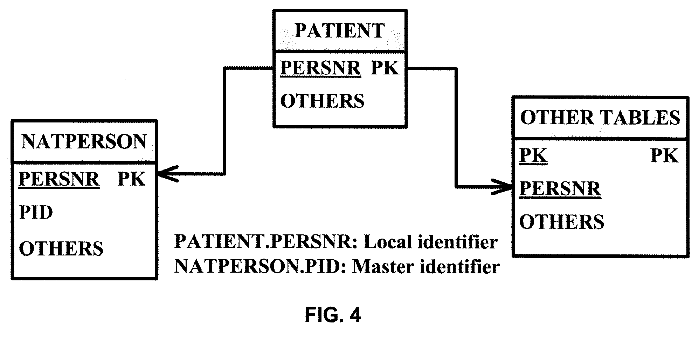

Fig 4. shows a sample data structure for identifiers

### Solution 1: Generate re-labeling statements with N3 query rule.

1. A set of source data which provides re-labeling relevant information is firstly generated (e.g. re-labeling_source_data.ttl in Table 11). It uses local identifiers to construct subject URIs, meanwhile also provides global identifiers, either expressed as numerals or literals.
2. A re-labeling query rule is created (e.g. re-labeling_query.n3q). It replaces a URI containing a local identifier with a new URI with corresponding global identifier.
3. Applying the re-labeling query rule (e.g.
   relabeling_query.n3q) created in step 2 to the statements (e.g. relabeling_source_data.ttl) generated in step1, a set of re-labeling statements (e.g. relabeling_statements.ttl) would be generated.
4. Applying the re-labeling statements (e.g.
   relabeling_statements.ttl) to data expressed with local identified URI (e.g. data expressed in table 1.), local identified URIs will be replaced with global identified URIs.

### Example

The example below uses the sample data structure displayed in Figure 4. The system uses PATIENT.PERSNR as its local identifier to link different tables. The master identifier is stored in the PID column of the NATPERSON table.

**Table 10: Sample data source query**

| |
|---|
| PREFIX patient: <http://www.agfa.com/w3c/orbis/orbis-schema/Patient#> |
| PREFIX natperson: <http://www.agfa.com/w3c/orbis/orbis-schema/Natperson#> |
| |
| ```
 CONSTRUCT {
     ?person
         natperson:pid
              ?personalIdentifier.
     ?patient
         patient:persnr
              ?person.
 } WHERE {
     ?person
         natperson:pid
              ?personalIdentifier.
     ?patient
         patient:persnr
              ?person.
 }
``` |

Executing the query stated in Table 10 returns a set of re-labeling source data, sample example of the returned data is shown in Table 11.

The patient reference
(<https://agfa.com/orbis/resource/Patient/10101>) uses local reference (PATIENT.PERSNR). The master identifier stored in the column PID of the NATPERSON table is retrieved as a number.

**Table 11: Sample relabeling data:relabeling_source_data**

| |
|---|
| <https://agfa.com/orbis/resource/Natperson/10101> |
| <http://www.agfa.com/w3c/orbis/orbis-schema/Natperson#pid> 8800123 . |
| <https://agfa.com/orbis/resource/Patient/10101> |
| <http://www.agfa.com/w3c/orbis/orbis-schema/Patient#persnr> |
| <https://agfa.com/orbis/resource/Natperson/10101>. |

**Table 12: Sample relabeling query rule:relabeling_query.n3q**

| |
|---|
| @prefix natperson: <http://www.agfa.com/w3c/orbis/orbis-schema/Natperson#> . |
| @prefix patient: <http://www.agfa.com/w3c/orbis/orbis-schema/Patient#> . |
| @prefix e: <http://eulersharp.sourceforge.net/2003/03swap/log-rules#>. |
| @prefix log: <http://www.w3.org/2000/10/swap/log#>. |
| @prefix str: <http://www.w3.org/2000/10/swap/string#>. |
| |
| ```
{
1 ?patient patient:persnr ?person.
2 ?person natperson:pid ?pid.
3 ?patient log:uri ?patientString.
4 (?patientString "/orbis" "") str:replace ?base.
5 (?patientString "/Patient/([^/]+)") str:scrape ?oid .
6 ?pid e:wwwFormEncode ?pid_e.
7 (?base ?oid ?pid_e) str:replace ?newUriString.
8 ?newUri log:uri ?newUriString.
}
=>
{
9 ?patient e:relabel ?newUri.
} .
``` |

The query rule stated in Table 12 would parse the patient URI expressed with local identifier (?patient) to construct a new URI with global identifier. Line 1 and 2 reads the input data. Line 3 generates a string from the patient URI, and Line 4 removes the data source indicator ('orbis') to serve as the base for the new URI. Line 5 retrieves the local identifier (in the sample data, it is 10101), Line 5 prepare the global identifier with WWW encode, and Line 6 replaces the local identifier with the global identifier. Line 8 creates a new URI from the modified literal (?newUeiString). The re-labeling statements is generated as described in Line 9.

Command to generate re-labeling statements:
eye --nope relabeling_source_data.ttl --query relabeling_query.n3q >relabeling_statements.ttl

**Table 13: Sample re-labeling statement:relabeling_statements.ttl**

| |
|---|
| #Processed by EYE-Autumn15 10191509Z josd |
| #eye --nope relabeling_data.ttl --query relabeling_query.n3q |
| PREFIX natperson: <http://www.agfa.com/w3c/orbis/orbis-schema/Natperson#> |
| PREFIX patient: <http://www.agfa.com/w3c/orbis/orbis-schema/Patient#> |
| PREFIX e: <http://eulersharp.sourceforge.net/2003/03swap/log-rules#> |
| PREFIX log: <http://www.w3.org/2000/10/swap/log#> |
| PREFIX string: <http://www.w3.org/2000/10/swap/string#> |
| |
| <https://agfa.com/orbis/resource/Patient/10101> e:relabel |
| <https://agfa.com/resource/Patient/8800123>. |
| #ENDS 0.093 [sec] 10=3/1 TC=1 TP=2 BC=0 BP=0 PM=0 CM=0 FM=0 AM=0 |

Table 13 shows a sample re-labeling statement with the sample data given in Table 11. Applying the re-labeling statements to the data in Table 1 will replace the patient URI expressed with a local identifier to a new one as stated in Table 13.

Command to apply re-labeling statements:
eye --nope data.ttl relabeling_statements.ttl --pass >result.ttl

**Table 14: Sample result: result.ttl**

| |
|---|
| #Processed by EYE-Autumn15 10191509Z josd |
| #eye --nope data.ttl relabeling_statements.ttl --pass |
| PREFIX natperson: <http://www.agfa.com/w3c/orbis/orbis-schema/Natperson#> |
| PREFIX patient: <http://www.agfa.com/w3c/orbis/orbis-schema/Patient#> |
| PREFIX e: <http://eulersharp.sourceforge.net/2003/03swap/log-rules#> |
| PREFIX log: <http://www.w3.org/2000/10/swap/log#> |
| PREFIX string: <http://www.w3.org/2000/10/swap/string#> |
| |
| <https://agfa.com/resource/Patient/8800123> a |
| <http://purl.bioontology.org/ontology/SNOMEDCT/116154003>. |
| <https://agfa.com/resource/Patient/8800123> |
| <http://aca.agfa.net/resource/ontology/healthcare#hasExamination> |
| <https://agfa.com/orbis/resource/LabResult/2454564>. |
| <https://agfa.com/resource/Patient/8800123> |
| <http://schema.org/identifier> 8800123 . |
| <https://agfa.com/orbis/resource/LabResult/2454564> |
| <http://aca.agfa.net/resource/ontology/healthcare#medicalCase> |
| <https://agfa.com/orbis/resource/Fall/12345>. |
| <https://agfa.com/orbis/resource/LabResult/2454564> |
| <http://schema.org/value> 9.60 . |
| #ENDS 0.062 [sec] 10=6/5 TC=0 TP=0 BC=0 BP=0 PM=0 CM=0 FM=0 AM=0 |

The results expressed in Table 14 can be merged with a global identified data from another data source without needing to take extra entailments.

### Solution 2: Generate re-labeling statements with a SQL script.

It is also possible to generate re-labeling statements with a SQL script, together with a template.

**Table 15: Sample SQL script**

| |
|---|
| ```
 SELECT
 '<{{BASE_URI}}/orbis/reference/Patient/' \| \|
 utl_url.escape(NATPERSON.PERSNR) \| \| '>' as "source",
 '<{{MASTER_BASE_URI}}/reference/Patient/' \| \|
 utl_url.escape(NATPERSON.PID) \| \| '>' as "mpi"
 FROM
 NATPERSON
``` |

Table 15 shows SQL scripts to generate source patent URIs (with local identifier) and target patient URIs (with global identifier). The placeholder {BASE_URI} and {MASTER_BASE_URI} allows to automatically adapt the URI construction to the site where it is deployed.

Executing the script in Table 15 will return a result set, and Table 16 is a template which processes the returned result set and generates re-labeling statements. The variables ?source and ?mpi in Table 16 correspond to the "source" and "mpi" stated in the SQL script in Table 15.

**Table 16: Sample SQL result template**

| |
|---|
| ```
prefix e: <http://eulersharp.sourceforge.net/2003/03swap/log-rules#>
 CONSTRUCT {
     ?source e:relabel ?mpi.
 }
 WHERE {
 }
``` |

Table 17 shows the re-labeling statement generated with this SQL based approach. As can be observed, it is the same as the statement generated with a query rule.

**Table 17: Sample relabeling statements from SQL**

| |
|---|
| <https://agfa.com/orbis/resource/Patient/10101> e:relabel |
| <https://agfa.com/resource/Patient/8800123>. |

The above-described re-labeling re-labeling is preferably performed on demand when exporting data from said EHR systems.

Having described in detail preferred embodiments of the current invention, it will now be apparent to those skilled in the art that numerous modifications can be made therein without departing from the scope of the invention as defined in the appending claims.

## Claims

1. A method to link instances expressed in at least two EHR systems by means of URIs constructed with different identifier sets comprising
processing RDF data of said instances to link said instances by generating re-labeling statements and applying said re-labeling statements to said RDF data to consolidate said URIs with different identifier sets to a single URI with a general master identity.

2. A method according to Claim 1 wherein said re-labeling statements are generated at runtime.

3. A method according to Claim 2 wherein said re-labeling statements are generated with N3 rules.

4. A method according to Claim 2 where said re-labeling statements are generated with SQL scripts, together with a template.

5. A method according to Claim 1 wherein said re-labeling is performed on demand when exporting data from said EHR systems.
